# EUROPÄISCHE PATENTSCHRIFT

(11) **EP 3 201 167 B1**
(45) Veröffentlichungstag und Bekanntmachung des Hinweises auf die Patenterteilung: **05.08.2020**
(21) Anmeldenummer: 15775409.4
(22) Anmeldetag: 23.09.2015
(51) Int. Cl.: B01D 3/34, C07C 51/48, C07C 51/44, C07C 51/42, C07C 57/04

(54) **VERFAHREN UND ANLAGE ZUR RÜCKGEWINNUNG VON ACRYLSÄURE**
METHOD AND SYSTEM FOR RECOVERING ACRYLIC ACID
PROCÉDÉ ET SYSTÈME DE RÉCUPÉRATION D'ACIDE ACRYLIQUE

(30) Priorität: 30.09.2014 DE 102014114193; 30.09.2014 US 201462057267 P
(43) Veröffentlichungstag der Anmeldung: 09.08.2017
(73) Patentinhaber: BASF SE, 67056 Ludwigshafen (DE)
(72) Erfinder: HAMMON, Ulrich, 68163 Mannheim (DE); WALTER, Thomas, 67454 Haßloch (DE); OTTENBACHER, Markus, 69259 Wilhelmsfeld (DE); HÜTTEN, Frank, 68165 Mannheim (DE)
(74) Vertreter: BASF IP Association
(86) Internationale Anmeldenummer: PCT/EP2015/071851
(87) Internationale Veröffentlichungsnummer: WO 2016/050582

(56) Entgegenhaltungen:
- DE-A1- 10 247 240
- DE-A1- 19 924 533
- DE-A1-102007 055 086

## Beschreibung

Die vorliegende Erfindung bezieht sich auf ein Verfahren und eine Anlage zur Rückgewinnung von Acrylsäure.

Acrylsäure ist eine bedeutende Grundchemikalie. Aufgrund ihrer sehr reaktionsfähigen Doppelbindung sowie der Säurefunktion eignet sich diese, insbesondere als Monomeres, zur Herstellung von Polymerisaten. Von der hergestellten Menge an Acrylsäure-Monomeren beispielsweise wird der größere Teil vor der Polymerisation verestert (z.B. zu Klebstoffen, Dispersionen oder Lacken). Nur der kleinere Teil wird direkt polymerisiert (z.B. zu "Superabsorbern"). Während im Allgemeinen bei der direkten Polymerisation der Acrylsäure Monomere hoher Reinheit benötigt werden, sind die Anforderungen an die Reinheit der Acrylsäure nicht so hoch, wenn diese vor der Polymerisation verestert wird.

Acrylsäure ist unter anderem durch heterogen katalysierte Gasphasen-Partialoxidation von C₃-Vorläufern der Acrylsäure mit molekularem Sauerstoff an im festen Aggregatzustand befindlichen Katalysatoren bei erhöhter Temperatur erhältlich. Unter dem Begriff C₃-Vorläufer werden solche chemischen Verbindungen zusammengefasst, die formal durch Reduktion von Acrylsäure erhältlich sind. Bei der Herstellung werden diese C₃-Vorläufer in gasförmigem Zustand, in der Regel mit inerten Gasen wie z.B. Stickstoff, CO₂, gesättigten Kohlenwasserstoffen und/oder Wasserdampf verdünnt, im Gemisch mit molekularem Sauerstoff bei erhöhten Temperaturen sowie gegebenenfalls erhöhtem Druck über übergangsmetallische Mischoxidkatalysatoren geleitet und oxidativ in ein Acrylsäure sowie Nebenkomponenten wie z.B. Furfurale, Benzaldehyd und Maleinsäureanhydrid enthaltendes Produktgasgemisch umgewandelt, aus welchem die Acrylsäure abgetrennt werden muss.

Bei der erhaltenen Acrylsäure handelt es sich nicht um ein reines Produkt, sondern um ein Gemisch, das neben Acrylsäure (in der Regel ≥ 90 %, oder ≥ 95 % des Gesamtgewichtes) noch typische Nebenprodukte der Gasphasenoxidation wie z.B. Wasser, niedere Aldehyde (z.B. Furfurale, Acrolein oder Methacrolein, Benzaldehyd), niedere Carbonsäuren (z.B. Essigsäure, Propionsäure) etc. sowie Oligomere der Acrylsäure enthält.

Ursächlich für die Entstehung von Oligomeren der Acrylsäure ist, dass in kondensierter Phase befindliche Acrylsäure durch reversible Michael-Addition an sich selbst sowie an das sich dabei bildende Dimere Acrylsäure-Oligomere (Michael-Addukte) bildet, ebenso wie durch radikalische Polymerisation entstehende Oligomere. Die Gegenwart von Wasser, dem unvermeidbaren Nebenprodukt einer gasphasenkatalytisch oxidativen Herstellung von Acrylsäure, sowie erhöhte Temperaturen fördern die Bildung von Oligomeren der Acrylsäure.

Da die jeweiligen Oligomere eine höhere Siedetemperatur als Acrylsäure aufweisen, reichern sie sich sowohl im Rahmen einer destillativen Abtrennung von Acrylsäure als auch bei einer fraktionierenden Kondensation des Produktgasgemisches einer gasphasenkatalytisch oxidativen Herstellung im Schwersiederbereich (z.B. in der Sumpfflüssigkeit) an.

Aus DE 199 24 533 A1 ist ein wie vorstehend beschriebenes Verfahren zur Herstellung von Acrylsäure bekannt, bei dem eine Grundabtrennung einer rohen Acrylsäure durch fraktionierende Kondensation des Produktgasgemisches der heterogen katalysierten Gasphasen-Partialoxidation vorgenommen wird. Eine Rückspaltung der im Auslass einer Quench-Flüssigkeit enthaltenen Acrylsäure-Oligomere soll dabei so integriert werden, dass die Laufzeit des Verfahrens, insbesondere der fraktionierenden Kondensation, im Wesentlichen nicht gemindert wird. Die Rückspaltung der Acrylsäure-Oligomere verfolgt dabei das Ziel, die Ausbeute an Wertprodukt zu erhöhen. DE 199 24 533 A1 sieht hierzu einen Umlaufreaktor für die Rückspaltung vor.

In DE 102 47 240 A1 wird ein sehr ähnliches Verfahren zur Herstellung von Acrylsäure beschrieben, bei dem ein Acrylsäure enthaltendes Produktgasgemisch durch direkte Kühlung mit einer Quench-Flüssigkeit zunächst abgekühlt und anschließend das abgekühlte Produktgasgemisch in eine mit trennwirksamen Einbauten ausgerüstete Kondensationskolonne geleitet wird. Aus der Kondensationskolonne wird rohe Acrylsäure entnommen und einer weiteren kristallisativen Reinigung zugeführt. Die bei dieser kristallisativen Reinigung anfallende Muttersäure wird hierbei vollständig in die Kondensationskolonne zurückgeführt. Aus dem Sumpf der Kondensationskolonne wird eine Acrylsäure-Oligomere enthaltende Sumpfflüssigkeit entnommen und als Quench-Flüssigkeit verwendet. Der beim Abkühlen des Produktgasgemisches nicht verdampfte Teil der Quench-Flüssigkeit wird über den Sumpf sowie gegebenenfalls über einen Wärmetauscher im Kreis geführt und als Auslass ein Teil der Quench-Flüssigkeit aus diesem Kreislauf ausschleust und einem Spaltgefäß zur Rückspaltung zugeführt. Die gasförmig entweichenden, Acrylsäure enthaltenden Spaltgase werden in den Kreislauf der Quench-Flüssigkeit oder in die Kondensationskolonne oder in den Kreislauf der Quench-Flüssigkeit und in die Kondensationskolonne rückführt, wobei die Spaltgase vor ihrer Rückführung einer Gegenstrom-Rektifikation und einer zumindest teilweisen Kondensation unterworfen werden, wobei die gebildete Kondensatmenge mindestens der zur Gegenstromrektifizierung notwendigen Rücklaufmenge entspricht.

Ferner beschreibt DE 10 2007 055 086 A1 ein Verfahren zur Herstellung von Acrylsäure, bei dem neben roher Acrylsäure an einem weiteren Seitenabzug Sauerwasser entnommen wird, das teilweise in die Kondensationskolonne zurückgeführt und teilweise durch Extraktion mit einem organischen Lösungsmittel unter Ausbildung eines Acrylsäure enthaltenden organischen Extrakts mit einem thermischen Trennverfahren weiterbehandelt wird. Die von dem Extrakt abgetrennte Acrylsäure wird in die Kondensationskolonne zurückgeführt oder der Weiterreinigung der rohen Acrylsäure zugeführt. Sofern die Acrylsäure aus dem organischen Extrakt mittels Strippen entfernt wird, kann das mit Acrylsäure beladene Strippgas dazu verwendet werden, um in einer zweiten Strippkolonne eine der Kondensationskolonne entnommene Sumpfflüssigkeit von dort noch enthaltender Acrylsäure ebenfalls freizustrippen bzw. Acrylsäureoligomere aus der Sumpfflüssigkeit rückzuspalten. D3 beschreibt nicht, dass ein Muttersäurestrom nach dem Temperieren in zwei Teilströme aufgeteilt werden kann, wovon einer als Rücklauf der Spaltkolonne zugeführt wird.

Die Verfahren nach dem Stand der Technik sind für sich genommen vorteilhaft und führen zu entsprechenden Ausbeuten an Wertprodukt, d.h. an Acrylsäure. Allerdings werden diese erhöhten Ausbeuten nur durch einen hohen apparativen Aufwand und Energieeinsatz ermöglicht.

Vor diesem Hintergrund ist es die Aufgabe der vorliegenden Erfindung, ein Verfahren zur Rückgewinnung von Acrylsäure anzugeben und eine entsprechende Anlage bereitzustellen, welche die Ausbeute an Wertprodukt gegenüber dem Stand der Technik weiter erhöhen, aber gleichzeitig eine effizientere Verfahrensführung im Hinblick auf den apparativen und energetischen Aufwand ermöglichen.

Die vorstehende Aufgabe wird in einem ersten Aspekt der vorliegenden Erfindung gelöst durch ein Verfahren zur Rückgewinnung von Acrylsäure, umfassend die Schritte
a) Teilen eines auf eine Temperatur von 50 °C bis 100 °C temperierten Muttersäurestroms aus einer Kristallisationsvorrichtung in einen ersten und einen zweiten Muttersäure-Teilstrom in Richtung einer Absorptionskolonne (201) und in Richtung einer Spaltkolonne (205), während der zweite Muttersäure-Teilstrom über eine Leitung (108) der Kondensationskolonne (201) zugeführt wird,
b) Zuführen des ersten temperierten Muttersäure-Teilstroms als Rücklauf zu dem obersten Boden der Spaltkolonne (205),
c) Zuführen zumindest eines Stripgasstroms unterhalb des untersten Bodens der Spaltkolonne (205),
d) Zuführen eines oligomere Acrylsäure umfassenden Nebenkomponentenstroms aus der Kondensationskolonne (201) zu einem mittleren Boden der Spaltkolonne (205),
e) Aufspalten zumindest eines Teils der oligomeren Acrylsäure aus dem Nebenkomponentenstrom in der Spaltkolonne (205) unter Erhalt von monomerer Acrylsäure,
f) Abtrennen der im Nebenkomponentenstrom enthaltenen Nebenkomponenten durch Gegenstromrektifikation in der aufgesetzten Spaltkolonne (205),
g) Abführen der monomeren Acrylsäure ohne Kondensation als Gasgemisch mit dem zugeführten Stripkreisgasstrom am Kopf der Spaltkolonne (205) und
h) Zuführen des Gasgemischs unterhalb des untersten Bodens der Kondensationskolonne (201).

Die vorstehende Aufgabe wird in einem zweiten Aspekt der vorliegenden Erfindung gelöst durch eine Anlage (1) zur Rückgewinnung von Acrylsäure umfassend
- eine Kondensationskolonne (201),
- eine Spaltkolonne (205),
- eine mit der Spaltkolonne (205) verbundene erste Leitung (101),
- eine die Kondensationskolonne (201) und die Spaltkolonne (205) verbindende zweite Leitung (102),
- eine dritte Leitung (103) zur Zufuhr eines Teilstroms der in der Kristallisation anfallenden Muttersäure zur Spaltkolonne (205),
- eine die Kristallisationsvorrichtung und die Absorptionskolonne (201) verbindende vierte Leitung (104), die in die dritte Leitung (103) und eine Leitung (108) zur Zufuhr eines Teilstroms der in der Kristallisation anfallenden Muttersäure zu der Kondensationskolonne (201) geteilt wird, und
- eine die Spaltkolonne (205) und die Kondensationskolonne (201) verbindende fünfte Leitung (105).

Die vorliegende Erfindung hat den wesentlichen Vorteil, dass mit dem erfindungsgemäßen Verfahren und der erfindungsgemäßen Anlage (1) bei der Acrylsäure-Herstellung anfallende Nebenkomponenten, insbesondere oligomere Acrylsäure, aufgespalten und mit verbesserter Effizienz in das Herstellungsverfahren zurückgeführt werden können, wodurch die Ausbeute an Wertprodukt, d.h. an Acrylsäure, und dessen Reinheit gesteigert werden kann.

Die vorliegende Erfindung wird nachstehend im Detail beschrieben.

Sofern in der nachfolgenden Beschreibung im Zusammenhang mit der erfindungsgemäßen Anlage (1) auch Verfahrensmerkmale genannt sind, beziehen sich diese vorzugsweise auf das erfindungsgemäße Verfahren. Ebenso beziehen sich gegenständliche Merkmale die im Zusammenhang mit dem erfindungsgemäßen Verfahren aufgeführt werden, vorzugsweise auf die erfindungsgemäße Anlage (1).

In einem ersten Aspekt der vorliegenden Erfindung wird die vorstehend genannte Aufgabe durch ein Verfahren zur Rückgewinnung von Acrylsäure gelöst. Das erfindungsgemäße Verfahren umfasst die Schritte a) bis h) und wird nachfolgend beschrieben.

In einem Schritt a) wird ein auf eine Temperatur von 50 °C bis 100 °C temperierter Muttersäurestrom aus einer Kristallisationsvorrichtung in einen ersten und einen zweiten Muttersäure-Teilstrom in Richtung einer Absorptionskolonne (201) und in Richtung einer Spaltkolonne (205) in zwei Teilströme geteilt, während der zweite Muttersäure-Teilstrom über eine Leitung (108) der Kondensationskolonne (201) zugeführt wird. "Temperiert" bedeutet im Sinne der vorliegenden Erfindung, dass der Muttersäurestrom, bzw. die Muttersäure-Teilströme nach dem Abführen aus der Kristallisationsvorrichtung auf eine Temperatur von 50 °C bis 100 °C, bevorzugt 60 °C bis 90°C, insbesondere 70 °C bis 80 °C erwärmt wurden. Die erfindungsgemäße Ausführung des Verfahrens ist dabei nicht auf eine bestimmtes Kristallisationsverfahren festgelegt, womit Acrylsäure durch teilweise Kristallisation, Abtrennung der ausgefrorenen Acrylsäure von der die Verunreinigungen enthaltenen Flüssigkeit (Muttersäure) und die durch Aufschmelzen der abgetrennten reinen Acrylsäurekristalle gereinigt wird. Hierbei kann z.B. eine Fallfilmkristallisation oder eine Suspensionskristallisation als Kombination aus Kühlscheibenkristallern und Waschkolonnen eingesetzt werden, wobei letztere Verfahrensvariante bevorzugt ist.

Mit "Muttersäure" (in vergleichbaren Schriften ggf. auch "Mutterlauge" genannt) wird in der vorliegenden Erfindung eine Lösung von Acrylsäure bezeichnet, die nach Abtrennung des Reinprodukts in einer Kristallisationsvorrichtung die in der Kristallisationsvorrichtung abgetrennten Verunreinigungen enthält, wobei auf die Acrylsäure in der Muttersäure ein Gewichtsanteil von ≥ 80 Gew.-% entfällt.

Ein erster temperierter Muttersäure-Teilstrom wird in Schritt b) auf den obersten Boden der Spaltkolonne (205) mit 45 Böden zugeführt. Die Spaltkolonne (205) ist in der vorliegenden Erfindung vorzugsweise mit Dual-Flow-Böden als trennwirksame Einbauten ausgerüstet. Ein zweiter temperierter Muttersäure-Teilstrom wird auf den Boden 18 der 75 Böden umfassenden Absorptionskolonne (201) geleitet.

Ein Schritt c) sieht das Zuführen zumindest eines Stripgasstroms unterhalb des untersten Bodens der Spaltkolonne (205) vor. Dabei ist der Stripgasstrom bevorzugt auf die Flüssigkeitsoberfläche der Sumpfflüssigkeit ausgerichtet. Der Stripgasstrom wird insbesondere als Kreisgas verwendet. Unter "Kreisgas" ist im Sinne der vorliegenden Erfindung ein Gas zu verstehen, welches in der Gasphasenoxidation zur Verdünnung der Edukte sowie der Aufnahme von Reaktionswärme dient und sich in der Gasphasen-Reaktion im Wesentlichen inert verhält. Das Kreisgas enthält im wesentlichen Stickstoff und im Konzentrationsbereich < 5 Vol.-% Sauerstoff, Wasserdampf, Kohlenstoffoxide und Mischungen davon und sehr geringe Mengen (< 0,8 Vol.-%) Ethylen, Ethan, Propen, Propan, Acrolein, Acrylsäure und Essigsäure.

In einem Schritt d) wird ferner ein oligomere Acrylsäure umfassender Nebenkomponentenstrom aus der Kondensationskolonne (201) zu einem mittleren Boden der Spaltkolonne (205) zugeführt. Bei diesem mittleren Boden handelt es sich insbesondere um einen Boden des Bodenbereichs 8 und 10.

In der Spaltkolonne (205) wird in einem Schritt e) zumindest ein Teil der oligomeren Acrylsäure aus dem Nebenkomponentenstrom unter Erhalt von monomerer Acrylsäure aufgespalten. Dies geschieht bevorzugt thermisch bei Temperaturen > 150 °C. Die Spaltung kann beschleunigt werden, wenn geringe Mengen von Natronlauge oder Aminen in den Sumpf der Spaltkolonne zugesetzt werden.

Schritt f) sieht das Abtrennen der im Nebenkomponentenstrom enthaltenen Nebenkomponenten durch Gegenstromrektifikation in der aufgesetzten Spaltkolonne (205) vor.

Die in Schritt e) erhaltene monomere Acrylsäure wird in einem Schritt g) ohne Kondensation als Gasgemisch zusammen mit dem zugeführten Stripkreisgasstrom am Kopf der Spaltkolonne (205) aus dieser abgeführt und anschließend in einem Schritt h) das Gasgemisch unterhalb des untersten Bodens der Kondensationskolonne (201) zugeführt.

Damit wird in vorteilhafter Weise die monomere Acrylsäure in den Prozess rückgeführt. Da die Acrylsäure in gasförmiger Form zugeführt wird und dort nicht erst verdampft werden muss, steht in der Kondensationskolonne (201) mehr Energie zur Trennung der Acrylsäure von Nebenkomponenten zur Verfügung. Die Kondensationskolonne (201) ist in der vorliegenden Erfindung vorzugsweise als Bodenkolonne ausgeführt. Sie ist bevorzugt mit Dual-Flow-Böden im unteren Bereich, mit Thormannböden im mittleren Bereich und mit Ventilböden im oberen Bereich ausgerüstet

Das erfindungsgemäße Verfahren weist den Vorteil gegenüber dem Stand der Technik auf, dass auf eine teilweise Kondensation der in der Spaltkolonne (205) ausgestrippten Acrylsäure zur Bedienung des Rücklaufs der Rektifikationskolonne verzichtet wird und anstelle des durch Kondensation der Brüden am Kopf der Spaltkolonne (205) erzeugten Rücklaufs ein Teil des in der Kristallisationsvorrichtung erzeugten Muttersäurestroms eingesetzt wird. Dadurch kann auf eine Kondensationseinheit am Kopf der Spaltkolonne (205) verzichtet werden, was den apparativen Aufwand reduziert. Zudem muss keine Kühlleistung zur Kondensation zur Verfügung gestellt werden. Weiterhin steht die über einen Sumpfwärmetauscher der Spaltkolonne (205) eingetragene Energie zusätzlich in der Kondensationskolonne (201) zur Verfügung, was die Trennung der Acrylsäure von Nebenkomponenten verbessert.

Ein weiterer Vorteil des vorliegenden Verfahrens ist, dass durch die erfindungsgemäße Verfahrensführung monomere Acrylsäure aus den jeweiligen Oligomeren in einer verbesserten Ausbeute zurückgewonnen und als Leichtsieder dem Gesamtprozess wieder zugeführt werden können. Unter "oligomerer Acrylsäure" werden insbesondere Dimere und Trimere der Acrylsäure verstanden. Gegenüber dem Stand der Technik reduziert sich der Aufarbeitungsverlust der Acrylsäure um 0,3 %. Unter "Aufarbeitungsverlust" wird in dieser Anmeldung der Anteil an Acrylsäure verstanden, der bezogenen auf die aus der Synthese zugeführten Acrylsäure nicht von den Nebenkomponenten abgetrennt und als Produkt gewonnen werden kann.

Das erfindungsgemäße Verfahren kann vorteilhafterweise mittels einer erfindungsgemäßen Anlage (1) ausgeführt werden, die nachstehend beschrieben wird.

In einer Weiterbildung des erfindungsgemäßen Verfahrens hat es sich als vorteilhaft herausgestellt, wenn der Muttersäurestrom zum Temperieren gegen einen Acrylsäurestrom aus einer Kondensationskolonne (201) geschaltet wird, insbesondere thermisch. Der Acrylsäurestrom wird bevorzugt als Zielprodukt über einen Seitenabzug der Kondensationskolonne (201) entnommen und im Wesentlichen einer Kristallisationsvorrichtung zugeführt. Dieser Acrylsäurestrom ist heiß (d.h. 95 °C bis 100 °C) und hochkonzentriert (d.h. 95 Gew.-% bis 98 Gew.-% Acrylsäure) und muss vor dem Eintritt in die Kristallisationsvorrichtung abgekühlt werden.

Erfindungsgemäß wird daher die in dem Acrylsäurestrom vorhandene Wärmeenergie mittels eines Wärmeübertragers auf den Muttersäurestrom übertragen und so in die beiden Muttersäure-Teilströme eingekoppelt. Damit steht diese Energie sowohl in der Absorptionskolonne (201) zur Auftrennung der Acrylsäure wie in der Spaltkolonne (205) zur Aufspaltung der dimeren Acrylsäure zur Verfügung, bzw. es muss weniger Energie über einen an der Spaltkolonne (205) vorgesehenen Sumpfwärmetauscher in die Spaltung eingetragen werden.

Nach einer bevorzugten Ausführungsform erfolgt Schritt h) indirekt, indem das Gasgemisch in eine Quench-Vorrichtung (203) zum Quenchen eines Acrylsäure enthaltenden Produktgasgemischs geleitet wird.

Der aus der Kristallisationsvorrichtung entnommene Muttersäurestrom umfasst vorzugsweise im Wesentlichen Acrylsäure sowie Anteile von Wasser und Essigsäure, insbesondere 90 Gew.-% bis 95 Gew.-% Acrylsäure, 3 Gew.-% bis 6 Gew.-% Wasser und 1 Gew.-% bis 2 Gew.-% Essigsäure sowie geringe Anteile (jeweils < 0,5 Gew.-%) an Formaldehyd, Propionsäure, Furfural, Maleinsäure und Diacrylsäure. Der Muttersäurestrom wird mit einer Temperatur knapp oberhalb der Kristallisationstemperatur von Acrylsäure (15 °C bis 20 °C) abgezogen und bevorzugt auf die entsprechende thermodynamische Gleichgewichtstemperatur am Ort der Einspeisestelle der Absorptionskolonne (201) von ca. 80 °C vorgewärmt, um eine möglichst effektive Trennung zu gewährleisten.

Ferner umfasst der Nebenkomponentenstrom vorzugsweise im Wesentlichen Acrylsäure, Diacrylsäure und Polyacrylsäure sowie Anteile von Maleinsäure, Benzoesäure, Benzaldehyd, Furfuralen und Wasser, insbesondere 50 Gew.-% bis 60 Gew.-% Acrylsäure oder Methacrylsäure, 20 Gew.-% bis 30 Gew.-% Diacrylsäure und 5 Gew.-% bis 10 Gew.-% Polyacrylsäure sowie 6 Gew.-% bis 9 Gew.-% Maleinsäure, 1 Gew.-% bis 2 Gew.-% Benzoesäure, 0,5 Gew.-% bis 1 Gew.-% Wasser und 0,5 Gew.-% bis 1 Gew.-% 4-Methoxyphenol, daneben geringe Anteile (jeweils < 0,5 Gew.-%) an Essigsäure, Furfural, Benzaldehyd, Phthalsäureanhydrid, Phenothiazin und Diacrylsäure. Der Nebenkomponentenstrom hat bevorzugt eine Temperatur von 100 °C bis 130 °C, insbesondere 105 °C bis 115 °C, um zum einen eine ausreichende Voreindickung der Sumpfflüssigkeit der Absorptionskolonne (201) vor Überführung in die Spaltkolonne (205) zu erreichen und zum anderen die Dimerenbildung im Sumpfbereich der Absorptionskolonne (201) zu begrenzen.

Außerdem kann der Stripgasstrom im Wesentlichen Stickstoff, Acrylsäure, Wasser und Sauerstoff sowie Anteile von Kohlendioxid und Essigsäure umfassen, insbesondere 80 Gew.-% bis 85 Gew.-% Stickstoff, 3 Gew.-% bis 5 Gew.-% Acrylsäure, 3 Gew.-% bis 5 Gew.-% Wasser, 3 Gew.-% bis 4 Gew.-% Sauerstoff, 2 Gew.-% bis 3 Gew.-% Kohlendioxid und 1 Gew.-% bis 2 Gew.-% Essigsäure sowie geringe Anteile (jeweils < 0,7 Gew.-%) an Kohlenmonoxid, Acrolein oder Methacrolein, Ameisensäure, Propen und Propan. Der Stripgasstrom hat bevorzugt eine Temperatur von 80 °C bis 90 °C, insbesondere ca. 85 °C.

Es hat sich für die Effizienz des erfindungsgemäßen Verfahrens, insbesondere für die Ausbeute an Wertprodukt, als vorteilhaft erwiesen, wenn in Schritt e) 60 % bis 95 %, insbesondere 85 % bis 90 %, der spaltbaren Komponenten des Nebenkomponentenstroms aufgespalten werden. Bevorzugt werden im Wesentlichen die Di- und Trimere der Acrylsäure aufgespalten. Was die Wertproduktausbeuten betrifft, sind höhere Spaltraten von 95 % vorteilhaft, jedoch technisch schwer beherrschbar, da bei Spaltausbeuten > 95 % das verbleibende Produkt zur massiven Feststoffbildung neigt und damit nur sehr schwer handhabbar ist.

In einem zweiten Aspekt der vorliegenden Erfindung wird die vorstehend genannte Aufgabe durch eine Anlage (1) zur Rückgewinnung von Acrylsäure gelöst. Die erfindungsgemäße Anlage (1) umfasst eine Kondensationskolonne (201) und eine Spaltkolonne (205).

Was unter der Kondensationskolonne (201) und der Spaltkolonne (205) im Sinne der vorliegenden Erfindung zu verstehen ist, wurde vorstehend in Bezug auf das erfindungsgemäße Verfahren schon beschrieben.

Die Anlage (1) umfasst ferner eine mit der Spaltkolonne (205) verbundene erste Leitung (101), die zumindest einen Gasstrom als Stripkreisgas zum Sumpfbereich der Spaltkolonne (205) zuführt. Eine zweite Leitung (102) verbindet die Kondensationskolonne (201) und die Spaltkolonne (205) und führt der Spaltkolonne (205) einen Nebenkomponentenstrom aus der Kondensationskolonne (201) zu.

Eine dritte Leitung (103) dient zur Zufuhr eines Teilstroms der in der Kristallisation anfallenden Muttersäure zur Spaltkolonne (205). Eine Kristallisationsvorrichtung und die Absorptionskolonne (201) werden durch eine vierte Leitung (104) verbunden, die in die dritte Leitung (103) und eine Leitung (108) zur Zufuhr eines Teilstroms der in der Kristallisation anfallenden Muttersäure zu der Kondensationskolonne (201) geteilt wird. Die Spaltkolonne (205) und die Kondensationskolonne (201) werden durch eine fünfte Leitung (105) verbunden.

Entsprechende Definitionen zu den Elementen der Anlage (1) und verwendeten Begriffen wurden vorstehend in Bezug auf das erfindungsgemäße Verfahren schon gegeben und gelten auch für diese Anlage (1).

Die Vorteile der erfindungsgemäßen Anlage (1) sind im Wesentlichen die gleichen wie für das vorstehend beschriebene erfindungsgemäße Verfahren. Die vorliegende Anlage (1) schafft die apparativen Voraussetzungen, um den Muttersäurestrom unter Erhalt zweier Muttersäure-Teilströme zu teilen und der Kondensationskolonne (201) sowie der Spaltkolonne (205) zuzuführen. Auf diese Weise wird keine Energie aus dem Gesamtprozess abgezogen, sondern verbleibt im Verfahren. Das Vorsehen zusätzlicher Wärmeübertrager zum Einkoppeln von externer Energie wird im Wesentlichen stark reduziert.

Zudem können im Stand der Technik in gattungsgemäßen Anlagen vorgesehene Anlagenteile und Vorrichtungen eingespart werden, beispielsweise Oberflächen- oder Sprühkondensatoren mit zugeordneten Pumpen und Wärmetauschern, desweiteren Einrichtungen zur Dosierung von Inhibitoren. Im laufenden Betrieb der Anlage können die Mengen an Inhibitoren zur Vermeidung von der Polymerisation von Acrylsäure reduziert werden, da über die Verwendung von Muttersäure als Rücklauf zur Spaltkolonne (205) bereits eine ausreichend stabilisierte Acrylsäure zu Verfügung steht.

In einer Weiterbildung der erfindungsgemäßen Anlage (1) umfasst diese ferner eine Quench-Vorrichtung (203) zum Quenchen eines Acrylsäure enthaltenden Produktgasgemischs, die in der fünften Leitung (105) zwischen der Spaltkolonne (205) und der Kondensationskolonne (201) angeordnet ist. Hiermit wird ein apparatives Mittel zur Verfügung gestellt, um das Gasgemisch aus der Spaltkolonne (205) und seine Temperatur zum Quenchen des heißen Produktgasgemischs in effizienter Weise auszunutzen.

Die erfindungsgemäße Anlage (1) ist insbesondere vorteilhaft, wenn sie in eine Gesamtanlage für die Acrylsäure- Herstellung integriert ist. Wie vorstehend beschrieben, kann die erfindungsgemäße Anlage (1) die Gesamteffizienz der Acrylsäure-Herstellung bei gleichzeitiger Verringerung des apparativen Aufwands erhöhen.

Weitere Ziele, Merkmale, Vorteile und Anwendungsmöglichkeiten ergeben sich aus der nachfolgenden Beschreibung von Ausführungsbeispielen der Erfindung anhand der Figur. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbeziehung. Es zeigt:

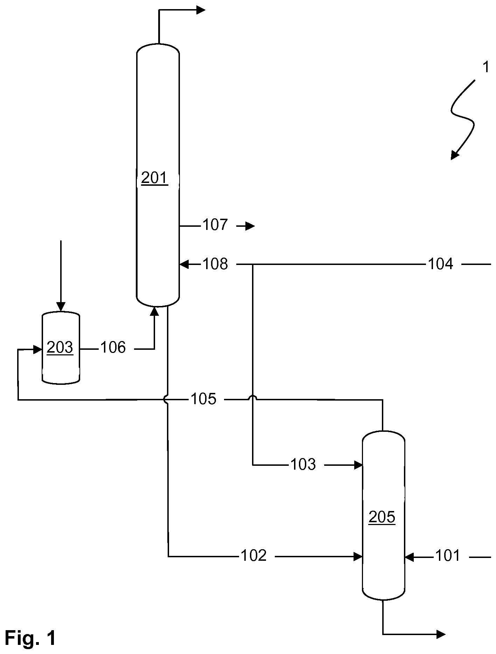

- Fig. 1: eine schematische Darstellung der erfindungsgemäßen Anlage 1 in einer Ausführungsform der Erfindung.

Figur 1 zeigt eine schematische Darstellung der erfindungsgemäßen Anlage 1 in einer Ausführungsform der Erfindung. Zentrale Elemente sind darin die Kondensationskolonne 201 und die Spaltkolonne 205. Aus einem nicht dargestellten Anlagenteil, in dem die heterogen katalysierte Gasphasen-Partialoxidation durchgeführt wird, wird ein heißer Produktgasstrom mit einer Temperatur von ca. 270 °C zugeführt. Dieser wird in eine Quench-Vorrichtung 203 geleitet, um mögliche Reaktionen seiner Bestandteile zu unterbinden und dessen Temperatur zu verringern. Ebenfalls der Quench-Vorrichtung 203 wird über eine fünfte Leitung 105 ein Gasgemisch aus der Spaltkolonne 205 mit einer Temperatur von ca. 95 °C zugeführt. Auf dieses Gasgemisch wird später genauer eingegangen.

Der der Quench-Vorrichtung 203 zugeführten Gase und Flüssigkeiten werden nach Austritt aus dieser in den Sumpfbereich der Absorptionskolonne 201 zugeführt. In dieser Absorptionskolonne 201 wird die hergestellte Acrylsäure durch Absorption aus dem Produktgemisch abgetrennt und über einen Seitenabzug aus der Absorptionskolonne 201 entnommen. Der abgezogene Acrylsäurestrom ist hochkonzentriert (ca. 97 % Acrylsäure) und hat eine Temperatur von ca. 99 °C. Dieser Acrylsäurestrom (auch als "rohe Acrylsäure" bezeichnet) wird über eine Reihe von Vorrichtungen, die hier nicht weiter beschrieben werden, einer Kristallisationsvorrichtung zugeführt, in der eine kristallisative Reinigung der Acrylsäure durchgeführt wird. Neben kristalliner hochreiner Acrylsäure verbleibt sog. Muttersäure in der Kristallisationsvorrichtung, die über eine Leitung 104 als Muttersäurestrom abgezogen wird.

Während nach dem Stand der Technik (siehe z.B. DE 102 47 240 A1) dieser Muttersäurestrom direkt einem unteren Boden einer Absorptionskolonne (vergleichbar mit Absorptionskolonne 201) zugeführt wird, besteht ein Merkmal der Erfindung darin, dass der Muttersäurestrom thermisch gegen den aus der Absorptionskolonne 201 abgezogenen Acrylsäurestrom gekoppelt wird. Der Muttersäurestrom hat zunächst eine Temperatur von ca. 20 °C und wird durch die thermische Kopplung auf ca. 93 °C erwärmt. Auf diese Weise kann die überschüssige Wärmeenergie des Acrylsäurestroms auf den Muttersäurestrom übertragen werden.

Der temperierte Muttersäurestrom wird in einen ersten und einen zweiten Muttersäure-Teilstrom geteilt. Während der zweite Muttersäure-Teilstrom über eine Leitung 108 der Absorptionskolonne 201 zugeführt wird, wird der erste Muttersäure-Teilstrom über eine dritte Leitung 103 als Rücklauf zu dem obersten Boden der Spaltkolonne 205 zugeführt und damit indirekt die Wärmeenergie des Acrylsäurestroms in die Spaltreaktion eingekoppelt.

Der Spaltkolonne 205 wird über eine erste Leitung 101 ein Stripgasstrom als Kreisgas aus einem nicht näher zu beschreibenden Anlagenteil unterhalb des untersten Bodens zugeführt. Dieser Stripgasstrom hat eine Temperatur von ca. 85 °C. Zu einem mittleren Boden der Spaltkolonne 205 wird aus dem Sumpf der Absorptionskolonne 201 über eine zweite Leitung 102 ein Nebenkomponentenstrom umfassend oligomere Acrylsäure mit einer Temperatur von ca. 109 °C zugeführt.

Der Nebenkomponentenstrom enthält Schwersieder wie Benzaldehyd, Furfural und Maleinsäure. Größter Bestandteil des Nebenkomponentenstroms sind jedoch Acrylsäure sowie deren Oligomere und Polyacrylsäure.

In der Spaltkolonne 205 werden diese Nebenkomponenten, insbesondere die Acrylsäure-Oligomere, rückgespalten und über deren Kopf als Leichtsieder- Fraktion zusammen mit dem Kreisgas abgezogen. Diese Leichtsieder-Fraktion bildet das Gasgemisch und wird über die Leitung 105 der Quench-Vorrichtung 203 zugeführt. Im Sumpf der Spaltkolonne 205 verbleiben insbesondere die schwersiedenden Anteile, die abgezogen und einer Entsorgung zugeführt werden.

In dem vorliegenden Ausführungsbeispiel wird durch das Rückspalten der Acrylsäure-Oligomere und deren Rückführung in die Kondensationskolonne 201 die Ausbeute an Acrylsäure als Wertprodukt deutlich gesteigert. Mit dem erfindungsgemäßen Verfahren und der erfindungsgemäßen Anlage 1 ist es möglich, eine Menge von 20,4 t/h Acrylsäure herzustellen.

Nachstehend wird eine konkrete Ausführungsform am Beispiel der Herstellung von Acrylsäure beschrieben.

### Beispiel (beschrieben wird der stationäre Zustand)

Aus einer heterogen katalysierten Gasphasenoxidation von Propylen der Reinheit "polymer grade" wurde ein eine Temperatur von 301 °C aufweisendes Produktgasgemisch der nachfolgenden Zusammensetzung erhalten:
12,168 Gew.-% Acrylsäure,
0,242 Gew.-% Essigsäure,
5,281 Gew.-% Wasser,
0,035 Gew.-% Ameisensäure,
0,18 Gew.-% Formaldehyd,
0,068 Gew.-% Acrolein,
0,005 Gew.-% Propionsäure,
0,003 Gew.-% Furfurale,
0,001 Gew.-% Allylacrylat,
0,0005 Gew.-% Allylformiat,
0,013 Gew.-% Benzaldehyd,
0,148 Gew.-% Maleinsäureanhydrid,
0,011 Gew.-% Benzoesäure,
0,011 Gew.-% Phthalsäureanhydrid,
2,126 Gew.-% CO₂,
0,658 Gew.-% CO,
0,08 Gew.-% Propan,
0,174 Gew.-% Propylen,
3,06 Gew.-% Sauerstoff und
75,728 Gew.-% Stickstoff.

Weitere Bestandteile werden nicht detektiert.

Das Produktgasgemisch (176.610 kg/h) wird in einem im Gleichstrom betriebenen Sprühkühler durch direkte Kühlung auf eine Temperatur von 120,1 °C abgekühlt. Die zur Direktkühlung verwendete Flüssigkeit ist ein Gemisch aus Sumpfflüssigkeit der Absorptionskolonne 201 und aus Schwersiederfraktion, die dem den Sumpfraum dieser Absorptionskolonne 201 abschließenden ersten Fangboden entnommen wird. Die Zusammensetzung der Sumpfflüssigkeit lautet:
35,59 Gew.-% Acrylsäure,
0,16 Gew.-% Essigsäure,
0,71 Gew.-% Wasser,
0,01 Gew.-% Ameisensäure,
<0,001 Gew.-% Formaldehyd,
0,01 Gew.-% Acrolein,
0,04 Gew.-% Propionsäure,
0,21 Gew.-% Furfurale,
0,001 Gew.-% Allylacrylat,
<0,001 Gew.-% Allylformiat,
0,68 Gew.-% Benzaldehyd,
10,56 Gew.-% Maleinsäureanhydrid,
0,683 Gew.-% Benzoesäure,
0,77 Gew.-% Phthalsäureanhydrid,
41,09 Gew.-% Diacrylsäure,
8,0 Gew.-% Polyacrylsäure (Michael-Addukte),
0,34 Gew.-% Phenothiazin,
0,82 Gew.-% MEHQ,
0,59 Gew.-% sonstige hochsiedende Bestandteile und
<0,001 Gew.-% Sauerstoff.

Die Schwersiederfraktion weist folgende Zusammensetzung auf:
86,62 Gew.-% Acrylsäure,
0,29 Gew.-% Essigsäure,
1,32 Gew.-% Wasser,
0,02 Gew.-% Ameisensäure,
0,002 Gew.-% Formaldehyd,
0,011 Gew.-% Acrolein,
0,09 Gew.-% Propionsäure,
0,42 Gew.-% Furfurale,
0,002 Gew.-% Allylacrylat,
0,001 Gew.-% Allylformiat,
1,03 Gew.-% Benzaldehyd,
8,39 Gew.-% Maleinsäureanhydrid,
0,03 Gew.-% Benzoesäure,
0,02 Gew.-% Phthalsäureanhydrid,
1,61 Gew.-% Diacrylsäure,
0,017 Gew.-% Phenothiazin,
0,07 Gew.-% MEHQ und
0,0004 Gew.-% Sauerstoff.

Die Menge der entnommenen Schwersiederfraktion beträgt 79.528 kg/h. Sie wird mit einer Temperatur von 105 °C entnommen und mit dieser Temperatur dem Sprühkühler zugeführt. Die Menge der der Absorptionskolonne 201 entnommenen Sumpfflüssigkeit beträgt 339.150 kg/h. Sie wird mit einer Temperatur von 120 °C entnommen. Dem Sprühkühler wird mit dieser Temperatur nur eine Menge von 336.690 kg/h zugeführt. 2.460 kg/h werden der Rückspaltung zugeführt.

Das bei der Direktkühlung resultierende Gemisch aus auf 126 °C abgekühltem Produktgasgemisch und Quenchflüssigkeit wird als solches in den Sumpf der Absorptionskolonne 201 geführt. Der Druck im Sumpfraum und im Sprühkühler beträgt 1,48 bar. Die Höhe der Absorptionskolonne 201 beträgt 54,3 m.

Der Innendurchmesser der Absorptionskolonne 201 beträgt im Bereich der Thormann-Böden 6,5 m und ansonsten 6,0 m.

2.460 kg/h der entnommenen Sumpfflüssigkeit werden der Spaltkolonne 205 (bestehend aus einem Zwangsumlaufentspannungsverdampfer und einer auf diesen nahtlos aufgesetzten Dual-Flow-Boden-Rektifikationskolonne) zugeführt. Die Anzahl der Dual-Flow-Böden beträgt 50. Ebenso wie die Absorptionskolonne 201 ist die Spaltkolonne 205 gegen die Umgebung isoliert. Der Innendurchmesser der Spaltkolonne 205 beträgt über alle Dual-Flow-Böden einheitlich 2,4 m. Ihre Höhe liegt bei 27 m. Die Dual-Flow-Böden sind in der Spaltkolonne 205 äquidistant (400 mm) angeordnet. Ihr Öffnungsverhältnis beträgt einheitlich 12 %. Von unten nach oben betrachtet beträgt der Lochdurchmesser der ersten acht Dual-Flow-Böden einheitlich 25 mm (Lochanordnung entsprechend strenger Dreiecksteilung) und der Lochdurchmesser aller nachfolgenden Dual-Flow-Böden liegt einheitlich bei 14 mm (Lochanordnung ebenfalls entsprechend strenger Dreiecksteilung). Die Zufuhr der der Rückspaltung zu unterwerfenden Sumpfflüssigkeit erfolgt auf den achten Dual-Flow-Boden.

In den Sumpf der Spaltkolonne 205 werden 19.999 kg/h von am Kopf der Absorptionskolonne 201 abgeführter, nachfolgend überhitzter und komprimierter Stripgasstrom zugeführt (Druck: 2,9 bar; Temperatur: 157 °C). Die Zusammensetzung des Stripgasstroms lautet:
0,269 Gew.-% Acrylsäure,
0,090 Gew.-% Essigsäure,
0,085 Gew.% Formaldehyd
2,689 Gew-.% Wasser,
0,009 Gew.-% Ameisensäure,
0,08 Gew.-% Acrolein,
0,001 Gew.-% Propionsäure,
0,001 Gew.-% Furfurale,
0,001 Gew.-% Allylformiat,
3,672 Gew.-% Sauerstoff,
2,517 Gew.-% CO₂,
0,779 Gew.-% CO,
0,095 Gew.-% Propan,
0,212 Gew.-% Propylen und
89,5 Gew.-% Stickstoff.

Dem Zwangsumlaufentspannungsverdampfer werden stetig 513.646 kg/h Flüssigphase mit einer Temperatur von 180 °C entnommen. Davon werden 512.997 kg/h mit einer Temperatur von 180 °C in den Zwangsumlaufentspannungsverdampfer rückgeführt. Die anderen 649 kg/h davon werden entgast und mit Methanol verdünnt einer Rückstandsverbrennung zugeführt.

Die im Zwangsumlaufentspannungsverdampfer gebildeten Spaltgase werden durch den zugeführten Stripgasstrom in die aufsitzende Rektifikationskolonne gefördert und steigen in dieser in absteigender Rücklaufflüssigkeit auf.

Aus dem Kopf der Rektifikationskolonne wird in einer Menge von 28.523 kg/h ein Gasgemisch (umfassend Stripgasstrom und Spaltgas) herausgeführt (Temperatur: 91 °C, Druck: 1,60 bar) und in den Sumpfraum der Absorptionskolonne 201 rückgeführt. Das rückgeführte Gasgemisch hat folgende Zusammensetzung:
28,8 Gew.-% Acrylsäure,
0,219 Gew.-% Essigsäure,
2,893 Gew.-% Wasser,
0,018 Gew.-% Ameisensäure,
0,081 Gew.-% Formaldehyd,
0,057 Gew.-% Acrolein,
0,041 Gew.-% Propionsäure,
0,028 Gew.-% Furfurale,
0,001 Gew.-% Allylacrylat,
0,001 Gew.-% Allylformiat,
0,004 Gew.-% Benzaldehyd,
0,004 Gew.-% Maleinsäureanhydrid,
2,54 Gew.-% Sauerstoff,
1,765 Gew.-% CO₂,
0,546 Gew.-% CO,
0,066 Gew.-% Propan,
0,149 Gew.-% Propylen und
62,788 Gew.-% Stickstoff.

Als Rücklaufflüssigkeit wird auf den obersten Boden der 50 Böden umfassenden Rektifikationskolonne 6.962 kg/h Muttersäure aus der Kristallisationsvorrichtung rückgeführt. Die Zusammensetzung der Mutterlauge lautet:
94,436 Gew.-% Acrylsäure,
0,596 Gew.-% Essigsäure,
3,788 Gew.-% Wasser,
0,044 Gew.-% Ameisensäure,
0,005 Gew.-% Acrolein,
0,156 Gew.-% Propionsäure,
0,127Gew.-% Furfurale,
0,003 Gew.-% Allylacrylat,
0,001 Gew.-% Allylformiat,
0,031 Gew.-% Benzaldehyd,
0,040 Gew.-% Maleinsäureanhydrid,
0,530 Gew.-% Diacrylsäure,
0,139 Gew.-% Polyacrylsäure (Michael-Addukte),
0,009 Gew.-% Phenothiazin,
0,022 Gew.-% MEHQ,
0,072 Gew.-% sonstige hochsiedende Bestandteile und
0,001 Gew.-% Sauerstoff.

In den Sumpfraum der Absorptionskolonne 201 ist ein Zentrifugaltropfenabscheider integriert, der verhindert, dass Tröpfchen der Sumpfflüssigkeit aus dem Sumpfraum heraus nach oben mitgerissen werden.

Der Sumpfraum der Absorptionskolonne 201 ist, wie bereits erwähnt, auf einer Kolonnenhöhe (wie alle Höhen vom Sumpfboden aus gerechnet) von 7,80 m durch einen ersten Fangboden (Sammelboden; Kaminböden mit 16 etwa gleichverteilten bedachten Kaminen; Kamindurchmesser: 600 mm; Kaminhöhe: 1 m) abgeschlossen.

Der Sammelboden ist doppelwandig mit 2° Gefälle nach Innen und mit zentraler Abzugstasse und Abzugsstutzen (DN-200) gestaltet. Der freie Gasquerschnitt beträgt ca. 30 %. Von diesem ersten Fangboden werden, wie bereits erwähnt, 83.559 kg/h Flüssigkeit entnommen und in den Sprühkühler geführt.

Die Sumpftemperatur beträgt 126 °C. Der Druck liegt bei 1,48 bar.

2,0 m oberhalb des ersten Fangbodens befindet sich der erste von zunächst 15 Dual-Flow-Böden. Diese Dual-Flow-Böden (Lochdurchmesser einheitlich 14 mm, Lochanzahl einheitlich 33.678, Öffnungsverhältnis einheitlich 18 %) sind äquidistant angebracht mit einem Bodenabstand von 380 mm. Die Durchtrittsöffnungen bestehen aus kreisrunden Öffnungen des einheitlichen Durchmessers von 14 mm, wobei der Stanzgrat in der Trennkolonne nach unten zeigte. Das Öffnungsverhältnis beträgt ca. 20 %. Die Anordnung der Mittelpunkte der Durchtrittskreise folgt einer strengen Dreiecksteilung. Der nächstliegende Abstand zweier Kreismittelpunkte liegt bei 30 mm.

Der fünfzehnte Dual-Flow-Boden ist als Verteilerboden gestaltet. Zu diesem Zweck enthält er zwei Einsteckrohre (DN-150) mit 40 Auslaufbohrungen (Durchmesser: 15 mm) je Einsteckrohr.

Die erste Serie von Dual-Flow-Böden wird mit einem zweiten Fangboden (Sammelboden; Kaminboden mit 16 ca. gleichmäßig verteilten bedachten Kaminen; Kaminhöhe ca. 1,70 m, zentrale Abzugstasse mit Abzugsstutzen (DN-250), freier Gasquerschnitt von - 30 %) abgeschlossen, der 1,50 m oberhalb des letzten Dual-Flow-Bodens untergebracht ist. Von diesem zweiten Fangboden wird bei 1,47 bar kontinuierlich rohe Acrylsäure mit einer Temperatur von 102 °C entnommen, die wie folgt zusammengesetzt ist:
97,076 Gew.-% Acrylsäure,
0,4200 Gew.-% Essigsäure,
1,614 Gew.-% Wasser,
0,021 Gew.-% Ameisensäure,
0,006 Gew.-% Formaldehyd,
0,004 Gew.-% Acrolein,
0,132 Gew.-% Propionsäure,
0,100 Gew.-% Furfurale,
0,003 Gew.-% Allylacrylate,
0,0006 Gew.-% Allylformiat,
0,025 Gew.-% Benzaldehyd,
0,031 Gew.-% Maleinsäureanhydrid,
0,543 Gew.-% Diacrylsäure,
0,007 Gew.-% Phenothiazin,
0,017 Gew.-% MEHQ und
0,0004 Gew.-% Sauerstoff.

53.766 kg/h der dem zweiten Fangboden entnommenen rohen Acrylsäure wird unmittelbar unterhalb des dem zweiten Fangboden nach oben folgenden Dual-Flow-Bodens in die Absorptionskolonne 201 rückgeführt.

91.152 kg/h der dem zweiten Fangboden entnommenen rohen Acrylsäure werden mehrstufig durch indirekten Wärmetausch (bevorzugt wärmeintegriert gegen in die Absorptionskolonne 201 rückzuführende Muttersäure) auf eine Temperatur von 29 °C abgekühlt. Dann werden der abgekühlten rohen Acrylsäure 1.694 kg/h des am Kopf der Absorptionskolonne 201 gewonnenen Sauerwassers zugefügt. Das resultierende Gemisch wird durch nochmaligen indirekten Wärmeaustausch auf 16,4 °C abgekühlt und dann in zwei bis drei Kühlscheibenkristallisatoren geführt.

Aus der Kristallisation werden nach teilweisem Kristallisieren, Abtrennen und Aufschmelzen in hydraulischen Waschkolonnen 20.973 kg/h an Reinacrylsäure der nachfolgenden Zusammensetzung entnommen:
99,8247 Gew-.% Acrylsäure,
0,1011 Gew.-% Essigsäure,
0,0210 Gew.-% Wasser,
0,0377 Gew.-% Propionsäure,
0,0001 Gew.-% Furfurale,
0,0001 Gew.-% Maleinsäureanhydrid,
0,0003 Gew.-% Diacrylsäure und
0,0150 Gew.-% MEHQ.

Sie eignet sich in hervorragender Weise zur Herstellung von Superabsorbern auf der Basis von Poly-Na-Acrylat.

In 834 kg/h der Reinacrylsäure werden 13 kg/h PTZ zur Herstellung einer Inhibitorlösung 1 gelöst. In 30 kg/h Inhibitorlösung 1 werden 19 kg/h MEHQ unter Bildung der Inhibitorlösung 2 gelöst.

Die in den hydraulischen Waschkolonnen abgetrennte Muttersäure wird zunächst in einen beheizbaren Sammelbehälter und von dort in einen Tank gefahren. Von diesem wird sie wärmeintegriert auf 90 °C erwärmt in einer Menge von 65.038 kg/h auf den fünfzehnten Dual-Flow-Boden der Absorptionskolonne 201 (von unten gerechnet) rückgeführt. Die Zusammensetzung dieser rückgeführten Muttersäure ist wie folgt:
94,436 Gew.-% Acrylsäure,
0,596 Gew.-% Essigsäure,
3,788 Gew.-% Wasser,
0,044 Gew.-% Ameisensäure,
0,005 Gew.-% Acrolein,
0,156 Gew.-% Propionsäure,
0,127Gew.-% Furfurale,
0,003 Gew.-% Allylacrylat,
0,001 Gew.-% Allylformiat,
0,031 Gew.-% Benzaldehyd,
0,040 Gew.-% Maleinsäureanhydrid,
0,530 Gew.-% Diacrylsäure,
0,139 Gew.-% Polyacrylsäure (Michael-Addukte),
0,009 Gew.-% Phenothiazin,
0,022 Gew.-% MEHQ,
0,072 Gew.-% sonstige hochsiedende Bestandteile und
0,001 Gew.-% Sauerstoff.

2,9 m oberhalb des zweiten Fangbodens befindet sich in der Absorptionskolonne 201 der erste von 21 weiteren Dual-Flow-Böden der bereits beschriebenen Art (Lochdurchmesser wieder einheitlich 14 mm, Lochanzahl jedoch einheitlich 32.020 und Öffnungsverhältnis einheitlich 17,4 %), die wieder äquidistant mit einem Bodenabstand von 380 mm angeordnet waren. Der letzte dieser 21 Dual-Flow-Böden ist mit Überlaufrinnen mit gezacktem Überlauf als Verteilerboden gestaltet.

800 mm oberhalb des letzten Dual-Flow-Bodens beginnt sich die Absorptionskolonne 201 konisch zu erweitern. 500 mm oberhalb des letzten Dual-Flow-Bodens endet diese Erweiterung bei einem Kolonneninnendurchmesser von 6,50 m.

Auf dieser Höhe, d.h., 1,50 m oberhalb des letzten Dual-Flow-Bodens, beginnt eine äquidistante (Bodenabstand = 500 mm) Anordnung von 28 konventionellen, einflutigen Thormann-Böden. Die Thormann-Böden sind derart ausgestaltet, dass über die Anordnung der Treibschlitze in den Hauben der Thormann-Böden in in Querstrom-Richtung aufeinanderfolgenden Rinnen jeweils eine zueinander entgegengesetzte Strömungsrichtung der Flüssigkeit erzeugt wird.

Das Öffnungsverhältnis der Thormann-Böden beträgt 14 %. Das Verhältnis von Kaminfläche zu Schlitzaustrittsfläche beträgt 0,8. Die Kaminhöhe und die Höhe des Ablaufwehrs beträgt 40 mm. Die Bodenfreiheit der Glocke (Abstand zwischen Unterkante Schlitz und Boden) beträgt 10 mm. Die Schlitzhöhe beträgt 15 mm. Der Winkel zwischen ausgestelltem Schlitz und Längskante der Haube beträgt 30 Grad. Die Länge der Längskante der Haube beträgt maximal 800 mm. Im Randbereich der Kolonne reduziert sich die Haubenlänge auf bis zu 200 mm aus Gründen der Anpassung an die Rundheit der Kolonne. Der Abstand zwischen zwei in Querstromrichtung auf einer Linie befindlichen Hauben beträgt 66 mm. Die Ablauffläche des Ablaufschachts beträgt 1,5 % bezogen auf die Querschnittsfläche des Bodens. Die Breite zwischen den beiden unteren Längsrändern einer Haube beträgt 64 mm.

Auf der Höhe des obersten Thormann-Bodens beginnt sich die Trennkolonne wieder konisch zu verengen. 700 mm oberhalb des obersten Thormann-Bodens ist diese Verengung abgeschlossen und der Kolonneninnendurchmesser wieder auf 6,00 m zurückgeschrumpft.

1,70 m oberhalb des obersten Thormann-Bodens befindet sich der dritte Fangboden (Sammelboden, Kaminboden mit 16 ca. gleichmäßig verteilten bedachten Kaminen, Kaminhöhe = 1,50 m).

Vom dritten Fangboden werden 533.818 kg/h Sauerwasser mit einer Temperatur von 68,6 °C und bei einem Druck von 1,24 bar entnommen. Die Zusammensetzung des Sauerwassers ist:
12,23 Gew.-% Acrylsäure,
4,00 Gew.-% Essigsäure,
78,72 Gew.-% Wasser,
0,70 Gew.-% Ameisensäure,
0,09 Gew.-% Formaldehyd,
0,01 Gew.-% Acrolein,
0,01 Gew.-% Propionsäure,
0,0016 Gew.-% Furfurale,
0,01 Gew.-% Allylformiat und
4,23 Gew.% Methylenglykol.

29.821 kg/h des entnommenen Sauerwassers (68,6 °C) werden zusammen mit der Inhibitorlösung 2 auf den obersten Thormann-Boden rückgeführt.

60 kg/h der Inhibitorlösung 2 werden (von unten betrachtet) auf den 19. Thormann-Boden rückgeführt (mit einer Temperatur von 25 °C und einem Druck von 1,10 bar).

6.828 kg/h des entnommenen Sauerwassers wurden der Verbrennung zugeführt. 392.000 kg/h des entnommenen Sauerwassers werden mit einer Temperatur von 36,6 °C auf den sechsten der nachfolgend zu beschreibenden Ventilböden (von unten gerechnet) rückgeführt.

277.000 kg/h des entnommenen Sauerwassers werden mit einer Temperatur von 31,4° C auf den obersten der nachfolgend zu beschreibenden Ventilböden rückgeführt.

2.300 mm oberhalb des dritten Fangbodens sind in der Kondensationskolonne in äquidistanter Anordnung (Bodenabstand = 500 mm) 11 zweiflutige Ventilböden angebracht. Die Höhe des Ablaufwehrs beträgt 35 mm. Das Öffnungsverhältnis liegt bei 18 % und die Summe der Ablaufflächen der Ablaufschächte von zwei aufeinanderfolgenden Ventilböden beträgt 10 % der Kolonnenquerschnittsfläche. Als Ventile wurden W12-Ventile der Fa. Stahl, DE, Viernheim verwendet. Der Druck am Kopf der Kolonne beträgt 1,2 bar.

Am Kolonnenkopf verlassen 169.164 kg/h Abgas mit einer Temperatur von 33,7 °C und der nachfolgenden Zusammensetzung die Absorptionskolonne 201:
0,27 Gew.-% Acrylsäure,
0,09 Gew.-% Essigsäure,
2,79 Gew.-% Wasser,
0,01 Gew.-% Ameisensäure,
0,08 Gew.-% Acrolein,
2,52 Gew.-% CO₂,
0,78 Gew.-% CO,
0,09 Gew.-% Propan,
0,21 Gew.-% Propylen
3,62 Gew.-% Sauerstoff und
89,54 Gew.-% Stickstoff.

In einem indirekten Wärmetauscher wird das Abgas auf 43 °C erwärmt und anschließend werden 94.691 kg/h dieses Abgases über einen Kreisgasverdichter als Verdünnungsgas in die Gasphasenoxidation und in die Rückspaltung geführt und 74.473 kg/h des Abgases werden der Verbrennung zugeführt.

### Vergleichsbeispiel

Das Vergleichsbeispiel wird im Wesentlichen wie das Beispiel mit dem Unterschied ausgeführt, dass keine Muttersäure auf den Kopf der Rektifikationskolonne gegeben wird und die gesamte Muttersäure auf den fünfzehnten Boden einer Absorptionskolonne geleitet wird und der Rücklauf der Rektifikationskolonne dadurch gebildet wird, dass am Kopf der Rektifikationskolonne durch einen aufgesetzten Sprühkondensator das aus der Rektifikationskolonne verlassende Gasgemisch auf 61 °C abgekühlt wird. Auf diese Art und Weise wurden 5.663 kg/h auskondensiert und auf den obersten Boden der Rektifikationskolonne aufgegeben. Dabei wurde dem Sprühkondensator kontinuierlich 60 kg/h einer Stabilisatorlösung, welche 0,98 Gew.% Phenothiazin gelöst in Reinprodukt enthielt, aufgegeben. Die auskondensierte Rücklaufflüssigkeit hatte dabei folgende Zusammensetzung:
95,10 Gew.% Acrylsäure,
0,05 Gew.% Furfurale,
0,02 Gew.% Acrolein,
0,03 Gew.% Ameisensäure,
0,52 Gew.% Essigsäure,
4,17 Gew.% Wasser,
0,1 Gew.% Propionsäure und
0,01 Gew.% Phenothiazin.

Alle anderen Verfahrensparameter wurden hierbei, soweit vorzugeben, konstant gehalten. Durch die Maßnahme erniedrigte sich die Sumpftemperatur der Absorptionskolonne auf 123 °C und der Rücklauf zur Absorptionskolonne ging auf 27.899 kg/h zurück. Im Abzug der Absorptionskolonne erhöhte sich der Essigsäuregehalt auf 0,8 Gew.-% und in Folge dessen wurde im Ausgang der Kristallisation eine geringere Reinheit der Acrylsäure von 99,75 Gew.-% mit 0,18 Gew.-% Essigsäure erhalten.

## Patentansprüche

1. Verfahren zur Rückgewinnung von Acrylsäure, umfassend die Schritte
a) Teilen eines auf eine Temperatur von 50 °C bis 100 °C temperierten Muttersäurestroms aus einer Kristallisationsvorrichtung in einen ersten und einen zweiten Muttersäure-Teilstrom in Richtung einer Absorptionskolonne (201) und in Richtung einer Spaltkolonne (205),
b) Zuführen des ersten temperierten Muttersäure-Teilstroms als Rücklauf zu dem obersten Boden der Spaltkolonne (205), während der zweite Muttersäure-Teilstrom über eine Leitung (108) der Kondensationskolonne (201) zugeführt wird,
c) Zuführen zumindest eines Stripgasstroms unterhalb des untersten Bodens der Spaltkolonne (205),
d) Zuführen eines oligomere Acrylsäure umfassenden Nebenkomponentenstroms aus der Kondensationskolonne (201) zu einem mittleren Boden der Spaltkolonne (205),
e) Aufspalten zumindest eines Teils der oligomeren Acrylsäure aus dem Nebenkomponentenstrom in der Spaltkolonne (205) unter Erhalt von monomerer Acrylsäure,
f) Abtrennen der im Nebenkomponentenstrom enthaltenen Nebenkomponenten durch Gegenstromrektifikation in der aufgesetzten Spaltkolonne (205),
g) Abführen der monomeren Acrylsäure ohne Kondensation als Gasgemisch mit dem zugeführten Stripkreisgasstrom am Kopf der Spaltkolonne (205) und
h) Zuführen des Gasgemischs unterhalb des untersten Bodens der Kondensationskolonne (201).

2. Verfahren nach Anspruch 1, wobei der Muttersäurestrom zum Temperieren gegen einen Acrylsäurestrom aus einer Kondensationskolonne (201) geschaltet wird.

3. Verfahren nach Anspruch 1 oder 2, wobei Schritt h) indirekt erfolgt, indem das Gasgemisch in eine Quench-Vorrichtung (203) zum Quenchen eines Acrylsäure enthaltenden Produktgasgemischs geleitet wird.

4. Verfahren nach einem der Ansprüche 1 bis 3, wobei der Muttersäurestrom im Wesentlichen Acrylsäure sowie Anteile von Wasser und Essigsäure umfasst.

5. Verfahren nach einem der Ansprüche 1 bis 4, wobei der Nebenkomponentenstrom im Wesentlichen Acrylsäure, Diacrylsäure und Polyacrylsäure sowie Anteile von Maleinsäure, Benzoesäure, Benzaldehyd, Furfuralen und Wasser umfasst.

6. Verfahren nach einem der Ansprüche 1 bis 5, wobei der Stripgasstrom im Wesentlichen Stickstoff, Acrylsäure, Wasser und Sauerstoff sowie Anteile von Kohlendioxid und Essigsäure umfasst.

7. Verfahren nach einem der Ansprüche 1 bis 6, wobei in Schritt e) 60 % bis 95 %, insbesondere 85 % bis 90 %, der spaltbaren Komponenten des Nebenkomponentenstroms aufgespalten werden.

8. Anlage (1) zur Rückgewinnung von Acrylsäure, umfassend
- eine Kondensationskolonne (201),
- eine Spaltkolonne (205),
- eine mit der Spaltkolonne (205) verbundene erste Leitung (101),
- eine die Kondensationskolonne (201) und die Spaltkolonne (205) verbindende zweite Leitung (102),
- eine dritte Leitung (103) zur Zufuhr eines Teilstroms der in der Kristallisation anfallenden Muttersäure zu der Spaltkolonne (205),
- eine die Kristallisationsvorrichtung und die Absorptionskolonne (201) verbindende vierte Leitung (104), die in die dritte Leitung (103) und eine Leitung (108) zur Zufuhr eines Teilstroms der in der Kristallisation anfallenden Muttersäure zu der Kondensationskolonne (201) geteilt wird, und
- eine die Spaltkolonne (205) und die Kondensationskolonne (201) verbindende fünfte Leitung (105).

9. Anlage nach Anspruch 8, ferner umfassend
- eine Quench-Vorrichtung (203) zum Quenchen eines Acrylsäure enthaltenden Produktgasgemischs, die in der fünften Leitung (105) zwischen der Spaltkolonne (205) und der Kondensationskolonne (201) angeordnet ist.

10. Anlage nach Anspruch 8 oder 9, wobei die Anlage (1) in eine Gesamtanlage für die Acrylsäure-Herstellung integriert ist.

## Claims

1. A process for recovering acrylic acid, which comprises the steps of
a) division of a mother acid stream which has been heated to a temperature of from 50°C to 100°C from a crystallization apparatus into a first mother acid substream and a second mother acid substream in the direction of an absorption column (201) and in the direction of a dissociation column (205),
b) feeding-in of the first heated mother acid substream as runback to the uppermost tray of the dissociation column (205), while the second mother acid substream is fed via a line (108) to the condensation column (201),
c) feeding-in of at least one stripping gas stream below the lowermost tray of the dissociation column (205),
d) feeding-in of a secondary component stream comprising oligomeric acrylic acid from the condensation column (201) to a middle tray of the dissociation column (205),
e) dissociation of at least part of the oligomeric acrylic acid from the secondary component stream in the dissociation column (205) to give monomeric acrylic acid,
f) removal of the secondary components comprised in the secondary component stream by countercurrent rectification in the superposed dissociation column (205),
g) discharge of the monomeric acrylic acid without condensation as gas mixture with the introduced circulating stripping gas stream at the top of the dissociation column (205) and
h) feeding-in of the gas mixture below the lowermost tray of the condensation column (201).

2. The process according to claim 1, wherein the mother acid stream is switched for heated against an acrylic acid stream from a condensation column (201) .

3. The process according to claim 1 or 2, wherein step h) is carried out indirectly by the gas mixture being introduced into a quenching apparatus (203) for quenching a product mixture comprising acrylic acid.

4. The process according to any of claims 1 to 3, wherein the mother acid stream comprises essentially acrylic acid and proportions of water and acetic acid.

5. The process according to any of claims 1 to 4, wherein the secondary component stream comprises essentially acrylic acid, diacrylic acid and polyacrylic acid and also proportions of maleic acid, benzoic acid, benzaldehyde, furfurals and water.

6. The process according to any of claims 1 to 5, wherein the stripping gas stream comprises essentially nitrogen, acrylic acid, water and oxygen and also proportions of carbon dioxide and acetic acid.

7. The process according to any of claims 1 to 6, wherein from 60% to 95%, in particular from 85% to 90%, of the dissociable components of the secondary component stream are dissociated in step e).

8. A plant (1) for recovering acrylic acid, which comprises
- a condensation column (201),
- a dissociation column (205),
- a first line (101) connected to the dissociation column (205),
- a second line (102) connecting the condensation column (201) and the dissociation column (205),
- a third line (103) for feeding a substream of the mother acid obtained in the crystallization into the dissociation column (205),
- a fourth line (104) which connects the crystallization apparatus and the absorption column (201) and is divided into the third line (103) and a line (108) for feeding a substream of the mother acid obtained in the crystallization to the condensation column (201) and
- a fifth line (105) connecting the dissociation column (205) and the condensation column (201).

9. The plant according to claim 8, which further comprises
- a quenching apparatus (203) for quenching a product gas mixture comprising acrylic acid, which is arranged in the fifth line (105) between the dissociation column (205) and the condensation column (201).

10. The plant according to claim 8 or 9, wherein the plant (1) is integrated into an overall plant for the preparation of acrylic acid.

## Revendications

1. Procédé pour la récupération d'acide acrylique, comprenant les étapes
a) partitionnement d'un flux d'acide mère tempéré à une température de 50 °C à 100 °C d'un dispositif de cristallisation en un premier et un deuxième flux partiel d'acide mère en direction d'une colonne d'absorption (201) et en direction d'une colonne de fractionnement (205),
b) introduction du premier flux partiel d'acide mère tempéré comme renvoi à l'étage le plus en haut de la colonne de fractionnement (205), pendant que le deuxième flux partiel d'acide mère est introduit par l'intermédiaire d'une conduite (108) dans la colonne de condensation (201),
c) introduction d'au moins un flux de gaz de strippage en dessous de l'étage le plus en bas de la colonne de fractionnement (205),
d) introduction d'un flux de composants secondaires comprenant de l'acide acrylique oligomérique de la colonne de condensation (201) à un étage médian de la colonne de fractionnement (205),
e) fractionnement d'au moins une partie de l'acide acrylique oligomérique du flux de composants secondaires dans la colonne de fractionnement (205) avec obtention d'acide acrylique monomérique,
f) séparation des composants secondaires contenus dans le flux de composants secondaires par rectification à contre-courant dans la colonne de fractionnement montée (205),
g) évacuation de l'acide acrylique monomérique sans condensation en tant que mélange de gaz avec le flux de gaz de circulation de strippage introduit au niveau de la tête de la colonne de fractionnement (205) et
h) introduction du mélange de gaz en dessous de l'étage le plus en bas de la colonne de condensation (201).

2. Procédé selon la revendication 1, le flux d'acide mère étant monté, pour le tempérage, contre un flux d'acide acrylique d'une colonne de condensation (201).

3. Procédé selon la revendication 1 ou 2, dans lequel l'étape h) est réalisée indirectement, par le fait que le mélange de gaz est conduit dans un dispositif d'extinction (203) pour l'extinction d'un mélange de gaz de produit contenant de l'acide acrylique.

4. Procédé selon l'une quelconque des revendications 1 à 3, le flux d'acide mère comprenant essentiellement de l'acide acrylique ainsi que des parties d'eau et d'acide acétique.

5. Procédé selon l'une quelconque des revendications 1 à 4, le flux de composants secondaires comprenant essentiellement de l'acide acrylique, de l'acide diacrylique et du poly(acide acrylique) ainsi que des parties d'acide maléique, d'acide benzoïque, de benzaldéhyde, de furfurals et d'eau.

6. Procédé selon l'une quelconque des revendications 1 à 5, le flux de gaz de strippage comprenant essentiellement de l'azote, de l'acide acrylique, de l'eau et de l'oxygène ainsi que des parties de dioxyde de carbone et d'acide acétique.

7. Procédé selon l'une quelconque des revendications 1 à 6, dans lequel dans l'étape e) 60 % à 95 %, en particulier 85 % à 90 % des composants fractionnables du flux de composants secondaires sont fractionnés.

8. Installation (1) pour la récupération d'acide acrylique, comprenant
- une colonne de condensation (201),
- une colonne de fractionnement (205),
- une première conduite (101) reliée à la colonne de fractionnement (205),
- une deuxième conduite (102) reliant la colonne de condensation (201) et la colonne de fractionnement (205),
- une troisième conduite (103) pour l'introduction d'un flux partiel de l'acide mère produit dans la cristallisation dans la colonne de fractionnement (205),
- une quatrième conduite (104) reliant le dispositif de cristallisation et la colonne d'absorption (201), qui est séparée en la troisième conduite (103) et une conduite (108) pour l'introduction d'un flux partiel de l'acide mère produit dans la cristallisation dans la colonne de condensation (201), et
- une cinquième conduite (105) reliant la colonne de fractionnement (205) et la colonne de condensation (201).

9. Installation selon la revendication 8, comprenant en outre
- un dispositif d'extinction (203) pour l'extinction d'un mélange de gaz de produit contenant de l'acide acrylique qui est disposé dans la cinquième conduite (105) entre la colonne de fractionnement (205) et la colonne de condensation (201).

10. Installation selon la revendication 8 ou 9, l'installation (1) étant intégrée dans une installation globale pour la préparation d'acide acrylique.
